(19) Europäisches Patentamt
European Patent Office
Office européen des brevets

(11) Publication number: 0 290 103 A2

# EUROPEAN PATENT APPLICATION

(21) Application number: 88200885.7

(22) Date of filing: 04.05.88

(51) Int. Cl.4: C07D 251/08 , C05C 9/02 , C05C 9/00

(30) Priority: 07.05.87 US 46648

(43) Date of publication of application: 09.11.88 Bulletin 88/45

(84) Designated Contracting States: BE DE ES FR GB IT NL

(71) Applicant: ARCADIAN CORPORATION
1 Gatehall Center
Parsappany New Jersey(US)

(72) Inventor: Hawkins, Edwin Francis
1238 Stoneleigh Drive
Baton Rouge Louisiana(US)

(74) Representative: van der Saag, Johannes et al
OCTROOIBUREAU VRIESENDORP & GAADE
P.O. Box 266
NL-2501 AW The Hague(NL)

## (54) Triazone fertilizer and method of making.

(57) A high-yield method producing novel water-soluble triazone compositions has a typical analysis having a yield of soluble triazone at about 48 percent, based on total weight of the reaction product, produced by a novel method in which ammonia/formaldehyde mole ratio is about 0.3, and in which percentage ammonia added and reacted during the initial reaction is about 4.5% by weight of total reactants, initial cooking being for about 45 minutes, followed by final cooking for about 10 minutes, both initial and final cooking being at about 90 degrees Centigrade, at a nitrogen content of about 28%, at an initial cooking-pH maintained immediately after ammonia addition, at about pH 9, and at a lower pH during the final cooking resulting from termination of adding further potassium hydroxide, the optimum mole ratio during the process, of reactants urea, formaldehyde and ammonia, for example, being about 0.9:1:00.30; otherwise, the method is a two stage-cooking procedure in which urea and/or particular substituted urea is reacted with particular aldehyde(s) and ammonia and/or particular primary amine(s). A number of novel soluble triazones have been produced at high yields, and found to be effective as fertilizers.

EP 0 290 103 A2

This invention is directed to a novel method for producing water-soluble triazones and to novel triazones and their use as foliar fertilizers.

## BACKGROUND TO THE INVENTION

Prior to this invention, the present inventor was granted United States patents nos. 4,554.005 and 4,559,102 granted Nov. 19, 1985 and July 8, 1986 respectively, directed to similar subject matter, namely novel water soluble triazone compositions and their methods of preparation.

It was desirable to obtain further novel water soluble triazone(s) of which it was not at all clear whether or not such compound could or could not be produced, never having existed before. A particular reason for uncertainty in these matters is founded on the fact that these water soluble triazone compounds cannot be readily isolated in the dry state. Their existence or ability to exist and be ineffective as such, depends upon many variables inclusive of particular percentages present of the water soluble triazone(s) and of particular percentages of each of several other components of the final reaction product.

Also, toxicity to foliage of novel triazone(s) is not reliably predictable, and likewise and accordingly no advance utility as a fertilizer, particular as a foliar fertilizer can be predicted heretofore.

It has also been desirable to substantially increase the yield of the water soluble triazone compositions of the above-noted prior patents and of the present novel water soluble triazone compositions, since the method of production heretofore has been considered undesirable due to lower yields of water soluble triazone.

In that regard, based on the emperical equation of reaction of formaldehyde and ammonia and urea to form water soluble triazone(s), the molar ratio of urea/formaldehyde/ammonia would reasonably be expected to be 1/2/1.

Also, based on such emperical reaction equation for producing water soluble triazone(s), it would be reasonable to expect yield to decrease as either urea or formaldehyde each increased in relative amount(s) with regard to the one quantity thereof relative to two quantities of formaldehyde based on the emperical formula.

## OBJECTS OF THE INVENTION

Accordingly, objects of the present invention include the obtaining of novel compositions and a novel method obtaining unexpectedly high yields of wayer soluble triazone(s) in the reaction product as compared to what was known or possible heretofore, and new use of novel compounds as fertilizers.

Other objects were to ascertain whether ranges of unexpectedly improved yields are obtainable for water soluble triazones utilizable as fertilizers, and whether equivalent reactants exist for water soluble triazones, and to determine which soluble triazones have utility as fertilizer and/or other utility, and to ascertain acceptable or preferred variations in the method of production of soluble triazones to obtain unexpected increase in yield and to avoid low yield for soluble triazone.

## SUMMARY OF THE INVENTION

Broadly, the invention may be described as a novel method obtaining unexpectedly high yield of triazone compositions of the novel triazone compositions noted below, and the novel triazone compositions themselves best produced by this new method. Additonally the invention includes the method of applying these novel triazone compositions as fertilizer by foliar or other method of application, foliar application being made possible by virtue of the water soluble nature of the novel triazone compositions. More particularly the inventive method novely has ascertained and utilized a critical caustic to be utilized in adjusting or maintaining particular pH during initial reaction, as well as having ascertained novel ranges and ratios and operating temperature ranges and periods of reaction, each of which critically effect and obtain unexpectedly high and improved yield(s) of water soluble triazone(s) in a two stage method of initial heating of reactants, followed by a second stage of heating as follows. The method of producing water soluble triazone compositions of this invention and of the prior above-noted patents includes, within aqueous media,

the initial cooking (reacting) of a urea-like component with an aldehyde and an ammonia-source reactant one of which is the particular triazone compound the preparation of which the novel method is directed. Accordingly, the triazone composition within the scope of this present and improved novel invention includes the following products in the broad and preferred ranges indicated. It should be noted that, as described before, the components of the final reacted product of the preceding novel method are all in and a part of an aqueous solution which, not being solid (dry) cannot be analyzed by prior typical nor known method for separating and identifying triazone compounds and percentages thereof, since to dry the present composition would destroy the required balance in percentages and the equilibrium, to produce undesirable products unstable and/or insoluble, to defeat the utility of the inventive triazone composition. Thus, as set-forth for example in inventor's prior patent United States 4,559,192 at column 8 thereof, lines 21 through 50, the analytical method utilizing a high performance liquid chromatography (designated HPLC) was and is utilized to separate reaction products and to identify the same, also using paper chromatographic separation.

Novel water-soluble triazone compounds newly produced as a part of novel triazone compositions produced by the method of this invention include:

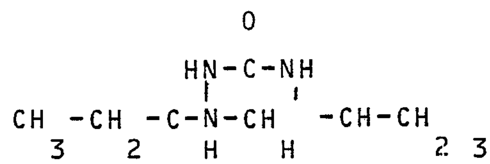
produced from urea,

propionaldehyde, ammonia; and likewise 1, 3, 4, 5, 6, pentaethyl triazone, and 1, 3, 4, 5, 6, pentamethyl triazone:

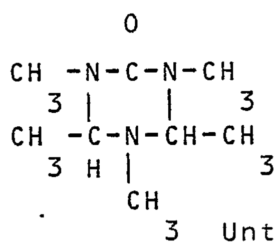
Until actual extensive experimentation by the present inventor, it was not possible to ascertain that these compounds could be produced nor that if produced that they would be soluble and stable (as to solubility and against breaking-up to form precipitates of other compounds), such as being discerned solely by the above-noted identification procedure and observation of prepared solutions thereof as to shelf life and stability at varying storage temperatures and periods of time.

Other generic novel triazones produced by this improved claimed method of this invention are as follow:

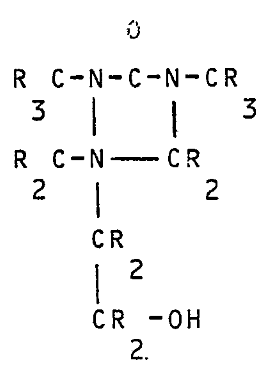

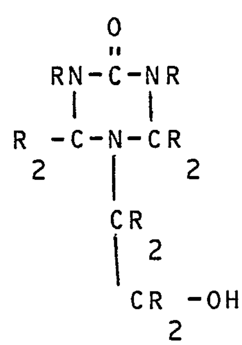

in which R is methyl or ethyl or non-carbon-substituted methyl or ethyl or hydrogen.

It has been previously noted that the production of high yield appears to be dependent on the particular caustic employed to maintain pH control during the initial cooking state, together with other process critical

limitations and parameters. However, additionally it surprisingly has been discovered thatfor the highest possible yields by this invention for the soluble triazone compounds, that the time of initiation of the pH control is highly critical for preferred results in one embodiment of this improved inventive method, based on experimentation by the inventor, which experimentation confirms trend as controlled by that factor.

The novel method includes the appropiate aqueous dilution of the reaction product, if any is desired or required to conform to conventional or desired manner of application, for the contemplated method of application to the soil, or sod, crop or other vegetation foliage, followed by appropriate deposit or application thereof.

However, an advantageous application arises by virtue of the high aqueous solubility and stability making possible easy use thereof by foliar spraying by either ground machines capable of spraying or by airplaines equipped to spray.

The novel methods of applying the fertilizer by mechanisms above-noted of this invention are limited to the application of the above-noted novel water-soluble triazone compositions that include one or more of the novel water-soluble triazone compounds described above.

## DETAILED DESCRIPTION

In the preceding novel method in the preparation of the triazone compositions containing the various above-described triazoned of the prededing patents and of the present invention, in the place of formaldehyde (where used for triazone products formed from aldehyde), there may be substituted in part or in whole substituted aldehyde(s) such as acetaldehyde and/or propionaldehyde. It is to be recognized that the aldehyde is dissolved (soluble) in water or an appropriate non-reactive organic of any desired or conventional nature, known in the art. In substitution in part or in whole, for the ammonia, any primary amine or substituted primary amine may be used such as methyl amine, monomethanol amine, dimethyl amine, amino methyl propanol, and the like.

It is in a combination of the present novel high aqueous solubility, high stability in solution, together with the low propensity to cause leaf-burn when applied directly to foliage that set these compounds and their use particularly in foliar application, apart from prior art. Propensity to cause foliar burning is not reliably predictable. It has been found by experimentation that the yield and the preferred yield of the present inventive methoa of producing, is critically affected by the timing and method of pH control, as evidenced by experiments discussed below, evidencing the effect of decreasing ammonia (relative to total weight of all reactants), on trends for lower or higher yields. As is typical, KOH is employed as commercially available aqueous 45% KOH, in experiments of this invention.

In a first set of experiments, apart from lesser amounts of caustic having been added, the pH control began at different points in time. In the runs where caustic addition began at the start of addition of urea/urea-formaldehyde solution --(85% solution), yields of soluble triazone were much higher than in contrast and comparison to runs in which an equivalent amount of total caustic added, was aaded rapidly at the beginning of the initial cook. It became conclusive that it is critical to add caustic only in a restricted or limited amount to the extent required for the maintaining of pH within an ascertained critical range of pH, and that too early addition of too much conclusively adversely affects yields of soluble triazone in the final product. In this series of first experiments, it was also observed that within a critical range, inclusion of lower amounts of ammonia and/or amine reactant results in lower yield of soluble triazone than in contrast to utilizing increased amount of the ammonia and/or amine reactant. Additionally, it was observed that significantly higher yields of soluble triazone was obtained in otherwise comparable runs, where the caustic employed was potassium hydroxide (KOH), as contrasted and compared to the equivalent runs utilizing sodium hydroxide (NaOH) as the caustic. Additionally, it was observed that within a critical range, that yield of soluble triazone increases as the amount of caustic employed increased to maintain elevated pH. A further run on a production scale of 180 pound batch served to establish that preceding observations continued to be true on a larger scale of commercial-type production.

In a second set of experiments, it was observed and demonstrated that to obtain high yield of triazone, there exists cooking time criticality for each of the separate initial and final cook periods, when within the critical parameters as to pH and amounts of reactants, ratios, and the like, and that within a ciritical range, an increase of amount of ammonia and/or amine reactant, relative to total weight of all reactants, results in an increase in yield of soluble triazone. It was also again observed that addition of KOH by gradual addition of moderate amounts required to merely maintain the optimum pH, resulted in higher yield of soluble triazone.

In a third series of experiments in which process parameters were substantially the same as the optimum ones ascertained in the second series the third set of experiments utilized components and conditions as follow:Ammonia/formaldehyde mole ratio --0.3;percentage anhydrous ammonia by weight of total reactants (& water) --4.9;initial cook --45minutes;second state or final cook --10 minutes; temperature of initial and final cooks --90 degrees Centigrade (C), ; us or ins 1:pH --9.0, plus or minus 0.3;and potassium hydroxide was the caustic utilized during the method. High yields resulted ranging between 42 and 48 percent --but predominantly between 47 and 48 percent, for this particular ammonia input and molar ratio.

In a further set of fourth experiments utilizing the same basic reactants and process parameters as for the third set of experiments, significantly higher yields of soluble triazone were obtained when ammonia was employed at even higher amounts relative to weight of total reactants.

In a fifth and sixth set of experiments, the reactant values and conditions of the process were as follow, except as otherwise noted:ammonia/formaldehyde molar ratio --0.3: percentage anhydrous ammonia --4.9;initial and final cooking temperatures --90 degrees C.;initial and final cooking periods and pH were baried, and the caustic employed was potassium hydroxide. It was observed that lower pH of 8.5, as contrasted to higher pH 9.0, and at low initial period of 15 minutes at pH 9.5, significantly lower triazone yield was obtained, as contrasted to pH 9.0 for initial heating period of 30 minutes with a final heating period of about 45 minutes which resulted in much higher yields. Excessively high pH of pH 9.5 resulted in lower yield at comparable heating periods.

In a seventh set of experiments, a comparison of runs using sodium hydroxide with other runs using potassium hydroxide as the caustic, were performed. It was ascertained that where the potassium hydroxide was employed, improved yields were obtained at the lower urea/formaldehyde weight ratio of 0.9 and at the lower ammonia/formaldehyde weight ratio of 0.32 and 0.31, when the weight percentage of KOH did not exceed 1.6.

In an eight series of experiments, it was observed that too high a level of potassium hydroxide, at 2.1, 2.1, 2.1, 2.4 and 2.2 respectively resulted in lower triazone yields, as contrasted to other runs at lower levels of potassium hydroxide. It was also noted that lower yields of triazone were obtained at excessively high amounts of ammonia, although limited-higher amounts of ammonia obtain higher yields of soluble triazone.

In a ninth series of experiments, it was observed that for one run thereof, the ratio of ammonia to formaldehyde at 0.4 far exceed allowable maximum critical limits, together with a too-low ammonia-formaldehyde ratio (at 0.6) at the near-upper pH-limit, and resulted in significantly lower triazone yield.

In a tenth series, the employment of massive urea at 63.7 percent by weight of total reactants even at a low final cooking period of time, resulted in a high yield of 45.8 percent for the soluble triazone, as adversely effected by a high KOH amount of 1.5. However, ammonia/formaldehyde ratio appeared to be lowered to a minimum of 0.34 --a run at 0.31 obtaining poor soluble triazone yield. Higher levels of urea, with other conditions within the inventive critical parameters, resulted in high soluble triazone yields.

In a fifteenth series, it was observed that preferred soluble triazone yields are obtained at cook temperature of 91 degrees Centigrade, making preferred critical temperature range from about 90.5 to about 91.5 degrees Centigrade when other inventive critical parameters and conditions are observed.

As noted above, it has not been possible to predict which water-soluble triazone products, if produced, would or would not be detrimental to plant life, particularly for a foliar fertilizer --a primary utility of the water-soluble triazones of this invention. In conformation of those facts, it was observed that there existed agromic diversity between the following triazones having the same molecular weight --namely 1.3. dimethyltriazone and 4.6. dimethyltriazone --both evaluated on turf in Florida for-St. Augustine and for Bermuda 419, as to leaf burn, colar rating, and in another test, evaluated as nitrogen source on bluegrass turf at Allentown, NJ, in comparison to urea at different pounds thereof, at maximum temperature between 90-95 degrees F. each day (for St. Augustine and Bermuda) applied on 5/5/85 with no rain until 5.20/85 at spray volume of 4 gal./100 sq. ft., plot size of 40 inches by 30 feet (100 sq. ft.). From all these tests, it was observed that the 4,6 dimethyl triazone is a safe, low burn, effective turf nitrogen source. In contrast, the 1,3 dimethyltriazone is an undesirable nitrogen source for turf because of its high burn potential exhibited. This latter product has characteristics more like a foliage desiccating agent, rather than or as opposed to a nitro-source fertilizer for which it has no utility.

## Claims

1. A method for producing high yields of soluble triazone, comprising in combination:employing initial reactants comprising a urea-type compound-source, an aldehyde-type compound-source, an ammonia-type compound-source, and a solubilizing amount of water for said reactants, in which the urea-type compound-source, relative to the aldehyde-like compound(s), has a first molar weight ratio ranging from about 0.65 to about 1.6, and in which the ammonia-type compound-source, relative to the aldehyde type compound-source, has a second molar ratio of from about 0.24 to about 0.40, the method comprising the steps of initially heating said initial reactants for an initial reaction period of cooking of from about 20 minutes to about 55 minutes while during said initial reaction period, adding from about 0.75 percent to about 2.5 percent by weight potassium hydroxide in an amount sufficient to maintain pH of said initial reactants at a pH of from about 8.6 to about 9.3, said heating being sufficient to maintain said initial reactants at a temperature of from about 87 degrees Centigrade to about 92 degrees Centigrade, to produce cooked reactants, and substantially immediately thereafter followed by a final heating of said cooked reactants at said temperature for a final cooking period of from about 7 minutes to about 35 minutes while substantially terminating addition of said caustic, provided that a sum of said initial reaction period and said final cooking period being up to about 70 minutes, and substantially permitting pH to vary by substantially terminating addition of further caustic during said final cooking period, reaction of said reactants during said initial period and said final cooking period being sufficient to form an aqueous solution of at least one soluble triazone of the formulae of the group consisting of:

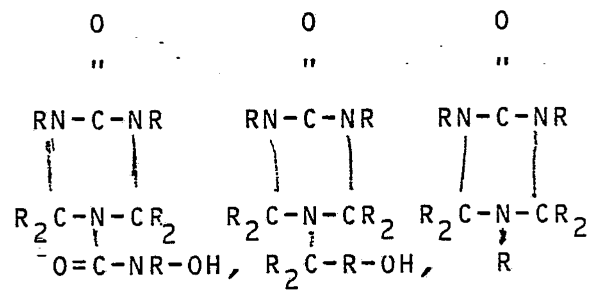

and

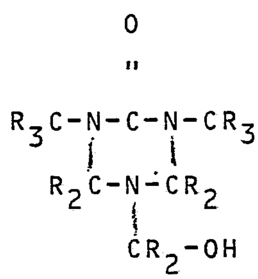

in which R is hydrogen, methyl, ethyl, methylol or ethylol.

2. A method according to claim 1, in which said first molar weight ratio is from about 0.72 to about 0.95, and in which molar weight ratio of said ammonia type compound-source/said aldehyde type compound-source ranges from about 0.28 to about 0.36, and in which said initial reaction period ranges from about 40 minutes to about 50 minutes, and in which said final cooking period is from about 10 minutes to about 20 minutes, said sum being up to about 60 minutes, and said caustic being added during said initial reaction period in an amount sufficient to maintain pH at from about pH. 8.7 to about 9.1, and in which said initial reaction period is at from about 90.5 degrees Centigrade to about 91.5 degrees Centigrade.

3. A method of claim 1, in which addition of said caustic during said initial reaction period, is commenced at substantially an end of adding ammonia type compound-source to others of said initial reactants, and addition of said caustic is added at any one or more points in time up to an amount sufficient to maintain said pH.

4. A soluble triazone compound entitled 1,3, dimethyl, 5, hydroxyethyl triazone having the emperical formula $C_7H_{15}N_3O_2$ and structural formula of:

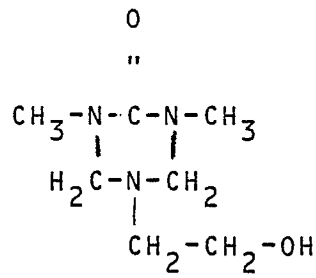

5. A soluble triazone compound entitled 4,6 dimethyl, 5, hydroxyethyl triazone having the emperical formula $C_7H_{15}N_3O_2$ and having a structural formula:

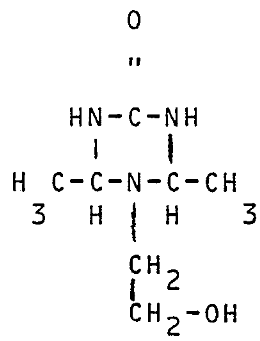

6. A soluble triazone compound entitled 4,6, diethyl, 5, hydroxyethyl triazone having the emperical formula $C_9H_{19}N_3O_2$ and having a structural formula:

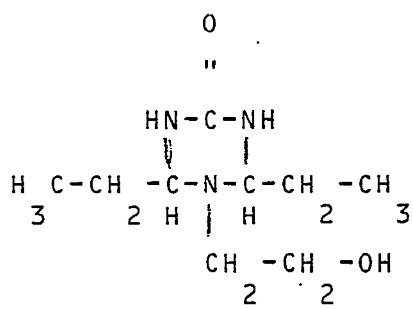

7. A soluble triazone composition comprising the soluble triazone composition in aqueous solution, identified and produced by the method of claim 1, having a soluble triazone yield of at-least about 40 percent by weight of total aqueous solution reaction product.

8. A soluble triazone composition of claim 7, in which said yield is at-least about 42 percent by weight of total aqueous solution reaction product.

9. A soluble triazone composition comprising the soluble triazone composition in aqueous solution, identified and produced by the method of claim 2, having a soluble triazone yield of at-least about 40 percent by weight of total aqueous solution reaction product.

10. A soluble triazone composition of claim 9, in which said yield is at-least 42 percent by weight of total aqueous solution reaction product.

11. A soluble triazone composition comprising the soluble triazone composition in aqueous solution, identified and produced by the method of claim 3.

12. A method of foliar spraying comprising foliar spraying an aqueous solution of a composition according to any of claims 7 through 11.

13. A method of foliar spraying comprising foliar spraying an aqueous solution of a soluble triazone of any of claims 4 through 6 and 14 through 26.

14. A method of claim 1, in which said ammonia-type compound source comprises at-least a major and predominant proportion of ammonia and employing said ammonia at a weight percentage ranging from about 3.1 percent to about 5.6 percent on a weight basis of all reactants and of water at time of reaction.

15. A method of claim 2, in which at-least a major and predominant proportion of said ammonia-type compound source consists essentially of ammonia, and employing said ammonia at a weight percentage ranging from about 3.1 percent to about 5.6 percent on a weight basis of all reactants and of water at time of reaction.

16. A method of claim 3, in which said ammonia-type compound source consists of ammonia, and employing said ammonia at a weight percentage ranging from about 3.1 percent to about 5.6 percent on a weight basis of all reactants and of water at time of reaction.

17. A method of claim 16, in which said ammonia type compound source is employed, relative to the aldehyde-like compound(s), in said second molar weight ratio ranging from about 0.25 to about 0.30.

18. A method of claim 2, in which said ammonia type compound source is employed, relative to the aldehyde-like compound(s) in said second molar weight ratio ranging from about 0.25 to about 0.30.

19. A water-soluble triazone compound entitled 1,3,diethyl, 5, hydroxyethyl triazone.

20. A water-soluble triazone compound entitled 1,3,4,5,6, pentamethyl triazone.

21. A water-solbule triazone compound entitled 1,3,dimethyl triazone.

22. A water-soluble triazone compound entitled 4,6,dimethyl triazone.

23. A water-soluble triazone compound entitled 4,6, diethyl triazone.

24. A water-soluble triazone compound entitled 1,3, diethyl triazone.

25. A water-soluble triazone compound entitled 1,3,4,5,6, pentaethyl triazone.

26. A triazone composition comprising the soluble triazone composition in aqueous solution, identified and produced by the method of any of claims 14 through 18.

27. A method of claim 1, including during said initial heating, adding said potassium hydroxide in an amount ranging between about 0.8 and about 1.8 percent by weight.

28. A method of claim 1, including during said initial heating, adding said potassium hydroxide in an amount ranging between about 0.8 and about 1.4 percent by weight.